Europäisches Patentamt

European Patent Office

Office européen des brevets

(11) Publication number: **0 368 373**
**A1**

## EUROPEAN PATENT APPLICATION

(21) Application number: **89202543.8**

(22) Date of filing: **09.10.89**

(51) Int. Cl.5: **A61K 31/57, A61K 31/56,**
**//(A61K31/57,31:565)**

(30) Priority: **13.10.88 NL 8802511**

(43) Date of publication of application:
**16.05.90 Bulletin 90/20**

(84) Designated Contracting States:
**AT BE CH DE ES FR GB GR IT LI NL SE**

(71) Applicant: **AKZO N.V.**
**Velperweg 76**
**NL-6824 BM Arnhem(NL)**

(72) Inventor: **de Jager, Evert**
**Mozartlaan 16**
**NL-5343 EL Oss(NL)**

(74) Representative: **Hermans, Franciscus G.M. et al**
**Patent Department AKZO N.V. Pharma Division P.O. Box 20**
**NL-5340 BH Oss(NL)**

(54) **Multi-phase contraceptive preparation.**

(57) Disclosed is a multi-phase contraceptive preparation having 22-30 dosage units. The preparation includes at least 2 phases. The first phase has 20-22 dosage units, each of which contains a contraceptively effective amount of progestogen and oestrogen. The second phase has 2 to 10 dosage units, which contain only a progestogen as the active contraceptive substance.

EP 0 368 373 A1

## Multi-phase contraceptive preparation

The invention relates to a multi-phase preparation containing substances with a contraceptive action in dosage units for oral administration having 22-30 dosage units in a specific sequence.

Oral contraceptives of the so-called combination type for cyclic use are generally known. Combination preparations of this type usually consist of 20-22 (usually 21) tablets of the same composition in respect of substances having a contraceptive action (e.g. progestogen and oestrogen or "estrogen"). One tablet is taken daily, after which a tablet-free period of 5-7 days usually follows. This regimen coincides with the natural female menstrual cycle of about 28 days. Menstruation occurs in the tablet-free period. After the tablet-free period a new cycle is started with the combination preparation. If desired, placebos may be administered during the tablet-free period. The advantage of placebo administration is that the daily administration of one tablet is not interrupted.

Multi-phase contraceptive preparations with which different quantities of progestogen and/or oestrogen are used in different phases of the cycle are also known.

See, for example, the following patents
US-A-3,795,734; US-A-3,939,264; US-A-3,957,982; US-A-3,969,502; US-A-4,066,757; US-A-4,378,356; US-A-4,390,531; US-A-4,530,839; US-A-4,544,554; DE-A-2,431,704; DE-A-3,341,638; DE-A-3,347,125; NL-B-169,681; and EP 148,724.

A goal of the multi-phase contraceptive preparations, generally of the combination type, is to achieve good control of the cycle while using smaller quantities of progestogen and/or oestrogen than are used in the monophase combination preparations.

Two-phase preparations of the so-called sequential and "normophase" type are also known with which exclusively oestrogenis dosed during a first phase and a combination of a progestogen and an oestrogen is dosed during a second phase. See, for example, US-A-3,409,721 and US-A-3,502,772.

In order to obtain adequate effectiveness with this type of preparation it is necessary in the first phase to use, per tablet, at least a quantity of oestrogen which corresponds in respect of activity to 0.050 mg ethinyl estradiol ("EE"). A lower oestrogen content corresponds with lower contraceptive reliability.

The known multi-phase contraceptive preparations illustrate the development which has already been underway for some time to develop oral contraceptives having the aim of reducing as far as possible the possible side effects of "the pill".

Possible side effects of the pill include cardiovascular venous and non-venous disorders, including thrombosis and hypertension; increased weight; breast cancer, and cancer of the cervix.

Commercially available monophase combination preparations with constant daily dosage have a total quantity of progestogen administered during a cycle of about 2.6 to about 3.2 mg, expressed in mg desogestrel or d-norgestrel (21 days x 0.125 mg/day or 21 days x 0.150 mg/day). The total quantity of oestrogen in these preparations is 0.6 and 1.0 mg, expressed in mg EE (21 x 0.030 mg or 21 x 0.050 mg). The larger quantity of progestogen is usually combined with a smaller quantity of oestrogen in such a preparation.

The lower limit for the total quantity of oestrogen per cycle would appear to be approximately 0.6 and 0.7 mg expressed in mg EE. If the oestrogen dose is too low, break-through bleeding and spotting take place, specifically in the second half of the cycle. These side effects could be countered by increasing the progestogen dose. However, the hormonal load on the body per unit time in the period in which active substances are administered becomes greater again.

In multi-phase preparations, the lowering of the total hormonal load is essentially effected by the low progestogen dosage at the start of the cycle, which is also the most desirable from the standpoint of cycle control. In practice, however, such a lowering is found to be at the expense of reliability. Increasing the progestogen dose at the start of the cycle brings us back to the tried concept of the monophase preparation with a constant daily dose of the progestogen and oestrogen combination.

A multi-phase contraceptive preparation having 22-30 dosage units has now been found which is a highly reliable contraceptive. The preparation includes at least two phases, the first of which consists of 20-22 dosage units which each contain both progestogen and oestrogen and a second phase having 2-10 dosage units which contain only progestogen as the active substance.

The invention relates to a multi-phase preparation containing contraceptive substances in dosage units for oral administration having 22-30 dosage units in a specific sequence. One dosage unit is administered daily during treatment and the sequence is determined in advance by the manner in which the dosage units are packaged. The preparation includes at least two phases, the first of which consists of 20-22, preferably 21, sequential dosage units, which contain both progestogenic and oestrogenic substances. It is possible, if

2

desired, for the first phase with dosage units of the combination type to contain various sub-phases with varying quantities of progestogen and/or oestrogen. The second phase consists of 2-10, preferably 7, sequential dosage units which contain only a progestogen as the active constituent, preferably in an amount per dosage unit which is lower than that in the last dosage unit of the first phase.

The amount of progestogen in the dosage units of the second phase can (for practical reasons) be kept constant. The second phase can, if desired, also consist of sub-phases with varying quantities of progestogen.

After administration of the final dosage unit of the phase(s) of the combination type, withdrawal bleeding will usually take place shortly afterwards because the oestrogen dosage is stopped. This bleeding may be delayed by several days, but this does not constitute an insurmountable drawback to starting with a new cycle of administration on the customary day in any case.

In principle, all substances having a progestogenic action are suitable as the progestogen in the preparation according to the invention, such as, for example, lynestrenol, desogestrel, norethisterone, norethisterone acetate, ethynodiol diacetate, dl-norgestrel, d-norgestrel, gestoden, norgestimate, norethynodrel and cingestol, and derivatives thereof, which are obtained by introduction of one or more double bonds, for example in the 14(15)-position, by substitution, for example with chlorine or methyl in, for example, the 7- or 11-position, or by preparation of functional derivatives, such as, for example, esters, in particular esters of alkane carboxylic acids having 1-12 C atoms, ethers, such as alkyl(1-4 C) ethers or acetals, such as ethylene diacetals or propylenediacetals.

Preferably, desogestrel, the 3-oxo-derivative of desogestrel, norgestimate, d-norgestrel or gestoden is used as progestogen. The greatest preference is, however, for desogestrel.

The customary oestrogens can be used as oestrogen in the first phase (the combination phase) of the preparation according to the invention, for example EE, mestranol, oestradiol, oestradiol esters and substituted derivatives hereof. The preference is for the orally effective oestradiol-$17\beta$-esters, but in particular for oestradiol itself (E2), the naturally occurring "female" steroid.

When quantities of progestogen or oestrogen are mentioned below, these relate to quantities of desogestrel as progestogen and quantities of ($17\beta$)-oestradiol as oestrogen unless expressly stated otherwise. These are then, however, always understood as meaning a quantity of another progestogen or oestrogen which is equivalent in respect of progestational or oestrogenic action.

If desired, different progestogens and/or oestrogens can be used in the different phases in the preparation according to the invention.

The quantity of progestogen in the tablets containing only a substance having a progestogenic action as the active constituent is usually between 0.020 mg and 0.125 mg, preferably between about 0.025 and about 0.050 mg. The quantity of progestogen per dosage unit in the second phase will preferably be lower the higher the number of dosage units and vice versa. The total quantity of progestogen in the second phase will be in the range between 0.120 mg and 0.250 mg. A practical quantity is, for example, 0.03 mg desogestrel per tablet for 6 or 7 tablets, which signifies a total quantity of 0.18-0.21 mg progestogen.

The total quantity of progestogen per cycle will be in the range of 1.5-3.5 mg, the major portion (about 1.3-3.3 mg) being administered during the combination phase and the remainder (about 0.12-0.25 mg) during the second phase. The distribution over the various dosage units of the combination phase is constant or phased and in the latter case can be taken identical to that in known 2- or 3-phase combination preparations.

In a preferred embodiment, the combination phase consists of several sub-phases, the progestogen content of the dosage units in each subsequent sub-phase being higher than that in the preceding sub-phase.

The total quantity of oestrogen in the combination phase of the preparation according to the invention is in the range from about 30 mg to about 75 mg. A practical quantity is, for example, 2.0 mg oestradiol per dosage unit which signifies a total quantity of 42 mg.

When dosage units are mentioned in the description (with the exception of the examples), these are understood as meaning oral dosage units such as tablets, pills, capsules, dragees and granules. The oral dosage units are obtained by mixing the desired quantities of the progestogen and the oestrogen with the conventional pharmaceutically acceptable auxiliaries, such as fillers, binders, disintegrating agents, colourings, flavourings and lubricants, and bringing the mixture into the form of a pharmaceutical shaped product, or filling capsules with the mixture.

A practical dosage form is the soft gelatin capsule, specifically in the case where an oestradiol $17\beta$-ester in a fluid lipoid substance, for example peanut oil or oleic acid, is used as the oestrogen. The active substances are then dissolved and/or dispersed in the lipoid fluid and the mixture is encapsulated in soft gelatin capsules in the conventional manner.

3

If desired, the preparation according to the invention can be supplemented with a number of placebos to bridge the period in which no active substances are administered, so that a regular daily swallowing of a dosage unit does not have to be interrupted.

It may be advisable to differentiate the placebos and the dosage units in the various phases from one another by different shaping and/or colour.

Preferably, days are indicated on the packs in which the preparation according to the invention is packaged, indicating at what time in the cycle the dosage unit corresponding to the indicated day must be taken.

The preparation can be packaged in a tube or box or in a so-called strip pack. In the case of a box, which can be round, rectangular or of another shape, the dosage units are sealed therein separately, usually around the circumference of the box, a series of day indications, which may or may not be adjustable, corresponding to the days on which each of the dosage units must be taken being given on the box.

Another practical type of packaging is the so-called strip pack or push-through pack, with which each dosage unit is sealed in a separate compartment and day indications or other indications which indicate the sequence in which the dosage units must be taken are given on the strip or the pack.

The invention is further illustrated with the aid of the following Examples.

## Example I

a) 2.381 g desogestrel and 3.968 g oestradiol 17$\beta$-cyclo-octylacetate were dissolved under sterile conditions in one litre of peanut oil. The solution obtained was encapsulated in the conventional manner in soft gelatin capsules with a capacity of 0.063 ml, so that each capsule contains 0.150 mg desogestrel and 0.250 mg oestradiol 17$\beta$-cyclo-octylacetate. The composition of the capsule wall was as follows: 69.8g by weight gelatin; 15.5% by weight glycerol; 13.7 % sorbitol; 0.44% by weight of the sodium salt of ethyl propyl parahydroxybenzoate and 0.56% by weight TiO$_2$.

b) A sterile solution of 0.476 g desogestrel in 1 litre of peanut oil was encapsulated in a similar manner in soft gelatin capsules with a capacity of 0.063 ml, so that each capsule contained 0.030 mg desogestrol.

c) Composition of the preparation:
1st phase: 21 capsules as obtained in example Ia);
2nd phase: 7 capsules as obtained in example Ib).

## Example II

Example I was repeated except that the oestradiol 17$\beta$-ester used in example Ia) was oestradiol 17$\beta$-(2-methyl)-decanoate in an amount of 7.936 g per litre, so that the capsules in the first phase contained, in addition to 0.150 mg desogestrol, 0.5 mg oestradiol 17$\beta$-(2-methyl)-decanoate.

## Example III

Composition of tablets in the first phase (21 tablets)
0.150 mg desogestrel
2.000 mg 17$\beta$-oestradiol
8.000 mg potato starch
2.400 mg polyvinylpyrrolidone
0.800 mg stearic acid
0.080 mg dl-$\alpha$-tocopherol
made up to
80.000 mg with lactose
in the 2nd phase (7 tablets)
0.030 mg desogestrel
8.000 mg potato starch
2.400 mg polyvinylpyrrolidone

0.800 mg stearic acid
0.800 mg silica
0.080 mg dl-α-tocopherol
made up to
80.000 mg with lactose.


## Example IV

Example III is repeated except that 30 g EE is used in place of 2.00 mg 17β-oestradiol.


## Example V

Composition of tablets in the first phase (21 tablets)
First sub-phase (7 tablets)
0.050 mg desogestrel
3.000 mg 17β-oestradiol
8.000 mg potato starch
2.400 mg polyvinylpyrrolidone
0.800 mg stearic acid
0.800 mg silica
0.080 mg dl-α-tocopherol made up to
80.000 mg with lactose.
2nd sub-phase (14 tablets)
0.150 mg desogestrel
2.000 mg 17β-oestradiol
8.000 mg potato starch
2.400 mg polyvinylpyrrolidone
0.800 mg silica
0.080 mg dl-α-tocopherol made up to
80.000 mg with lactose.
in the 2nd phase (7 tablets)
0.030 mg desogestrel
8.000 mg potato starch
2.400 mg polyvinylpyrrolidone
0.800 mg stearic acid
0.800 mg silica
0.080 mg dl-α-tocopherol
made up to
80.000 mg with lactose.


## Example VI

Composition of tablets
As in example V except that 0.035 mg EE is used in the first sub-phase and 0.030 mg EE is used in the second sub-phase.


## Example VII

Composition of tablets
In the first phase (21 tablets)

1st sub-phase (7 tablets)
0.050 mg desogestrel
3.000 mg 17β-oestradiol
8.000 mg potato starch
2.400 mg polyvinylpyrrolidone
0.800 mg stearic acid
0.800 mg silica
0.080 mg dl-α-tocopherol
made up to
80.000 mg with lactose.
2nd sub-phase (7 tablets)
0.100 mg desogestrel
2.000 mg 17β-oestradiol
and in other respects identical to the composition in the first sub-phase;
3rd sub-phase (7 tablets)
0.150 mg desogestrel
1.500 mg 17β-oestradiol
and in other respects identical to the composition in the preceding sub-phases; in the 2nd phase (7 tablets)
0.030 mg desogestrel and in other respects identical to the composition in the preceding phases.

Example VIII

As in Example VII, the first phase, however, consisting of 22 tablets (by making the third sub-phase consist of 8 tablets) and the second phase consisting of 6 tablets.

Example IX

Composition of dragees
in the lst phase (21 dragees)
1st sub-phase (11 dragees)
0.050 mg d-norgestrel
2.000 mg 17β-oestradiol
15.000 mg maize starch
2.000 mg polyvinylpyrrolidone
1.500 mg talc
made up to
80.000 mg with lactose;
2nd sub-phase (10 dragees)
0.125 mgd-norgestrel
2.000 mg 17β-oestradiol
and in other respects identical to the composition in the first sub-phase; in the 2nd phase (2 dragees) 0.125
mg d-norgestrel and in respect of auxiliaries identical to the composition in the preceding phases.

Example X

Composition of dragees which in respect of auxiliaries are identical to those in Example IX.

| 6 dragees: | 0.050 mg d-norgestrel | |
| | 2.000 mg 17β-oestradiol | (1st phase) |
| 5 dragees: | 0.075 mg d-norgestrel | |
| | 2.500 mg 17β-oestradiol | |
| 10 dragees: | 0.125 mg d-norgestrel | |
| | 2.000 mg 17β-oestradiol | |
| 3 dragees: | 0.070 mg d-norgestrel | (2nd phase) |
| 2 dragees | 0.050 mg d-norgestrel | |

Example XI

As for Example X, gestoden, however, being used in an equivalent amount in place of d-norgestrel.

Reference herein to specific embodiments or examples is not intended to limit the scope of the invention which is defined by the appended claims.

**Claims**

1. A multi-phase preparation containing substances having a contraceptive action in dosage units for oral administration in which there are 22-30 dosage units in a specific sequence, one of which is administered daily during the period of administration and the sequence is determined in advance by the manner in which the dosage units are fit into the packaging of the preparation, which preparation comprises: a first phase consisting of 20-22, preferably 21, sequential dosage units, which dosage units contain both progestogenic and oestrogenic substances, it being possible, if desired, for this first phase with dosage units of the combination type to consist of various sub-phases with varying quantities of progestogen and/or oestrogen; and/or a second phase consisting of 2-10, preferably 7, sequential dosage units which contain only a progestogen as active constituent.

2. The preparation according to claim 1, wherein the progestogen is desogestrel.

3. The preparation according to claim 1 or 2, wherein the quantity of progestogen per dosage unit in the second phase is lower than the quantity of progestogen in the last dosage unit of the first phase.

4. The preparation according to claim 1, 2, or 3, wherein the quantity of progestogen in the dosage units of the second phase is from about 0.020 to about 0.125 mg, preferably 0.025-0.050 mg, expressed in mg desogestrel per dosage unit.

5. The preparation according to claims 1-4, wherein the total quantity of progestogen in the second phase is between 0.120 mg and 0.250 mg, expressed as mg desogestrel.

6. The preparation according to claims 1-5, wherein the total quantity of progestogen in both phases is in the range of about 1.5 to about 3.5 mg.

7. The preparation according to claims 1-6, wherein 17β-oestradiol (E2) is used as an oestrogenic substance.

8. The preparation according to claims 1-7, wherein the total quantity of oestrogen is in the range of from about 30 mg to about 75 mg, expressed as mg oestradiol.

9. The preparation according to claims 1-8, wherein the second phase consists of sub-phases with different quantities of progestogen.

10. The preparation according to claims 1-9, consisting of a first phase of 21 dosage units, each containing 0.150 desogestrel and 2 mg E2 as active constituents, and a second phase of 7 dosage units, each containing 0.030 mg desogestrel as an active constituent.

11. The preparation according to claims 1-9, consisting of a first phase of 21 dosage units which, in turn, consist of a first sub-phase of 7 dosage units, each containing 0.050 mg desogestrel and 3 mg E2, a second sub-phase of 7 dosage units, each containing 0.100 mg desogestrel and 2 mg E2, and a third sub-phase of 7 dosage units, each containing 0.150 mg desogestrel and 1.5 mg E2 as active constituents, and a second phase of 7 dosage units, each containing 0.030 mg desogestrel as an active constituent.

12. A treatment for controlling fertility in a fertile woman, said treatment comprising: orally administering to the woman, on a daily basis for 20 to 22 sequential days, first dosage units, each of said first dosage units comprising an admixture of progestogenic and oestrogenic substances; and

orally administering to the woman, on a daily basis for 2 to 10 sequential days, second dosage units consisting essentially of a progestogenic substance;

said progestogenic and oestrogenic substances being present in said dosage units in amounts sufficient to act as a contraceptive in the woman during the time of treatment when administered on a daily basis.

13. The treatment of claim 12 wherein a first dosage unit is administered on a daily basis for each of 21 sequential days.

14. The treatment of claim 13 wherein a second dosage unit is administered to said woman on a daily basis for each of 7 sequential days.

15. The treatment of claim 14 wherein the amount of progestogen per second dosage unit is lower than the amount of progestogen in the last administered first dosage unit.

16. The treatment of claim 15 wherein the amount of progestogen in each of the second dosage units contains from about 0.020 to about 0.125 milligrams, expressed in milligrams desogestrel, per dosage unit.

17. The treatment according to claim 16 wherein $17\beta$-oestradiol is the oestrogenic substance.

European Patent Office

**PARTIAL EUROPEAN SEARCH REPORT**
which under Rule 45 of the European Patent Convention
shall be considered, for the purposes of subsequent
proceedings, as the European search report

Application number

EP 89 20 2543

## DOCUMENTS CONSIDERED TO BE RELEVANT

| Category | Citation of document with indication, where appropriate, of relevant passages | Relevant to claim | CLASSIFICATION OF THE APPLICATION (Int. Cl.⁴) |
|---|---|---|---|
| X | FR-A-2 182 992 (SYNTEX)<br><br>* Claims 1-19; examples XII,XIII; page 5, paragraph 2; page 13 * | 1,4,6, 7,9,10 | A 61 K 31/57<br>A 61 K 31/56//<br>(A 61 K 31/57,<br>A 61 K 31:565) |

----

**TECHNICAL FIELDS SEARCHED (Int. Cl.⁴)**

A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-11
Claims searched incompletely:
Claims not searched: 12-17
Reason for the limitation of the search:

Art. 52(1); Art. 57

| Place of search | Date of completion of the search | Examiner |
|---|---|---|
| THE HAGUE | 14-02-1990 | PEETERS |